# EUROPÄISCHE PATENTANMELDUNG

(11) **EP 1 203 563 A2**
(43) Veröffentlichungstag der Anmeldung: **08.05.2002**
(21) Anmeldenummer: 01124393.8
(22) Anmeldetag: 25.10.2001
(51) Int. Cl.: A61B 5/15

(54) **Analytisches Hilfsmittel mit integrierter Lanzette**

(30) Priorität: 31.10.2000 DE 10053930; 16.01.2001 DE 10101658
(71) Anmelder: Roche Diagnostics GmbH, 68305 Mannheim (DE); F. HOFFMANN-LA ROCHE AG, 4002 Basel (CH)
(72) Erfinder: Haar, Hans-Peter, 69168 Wiesloch (DE); List, Hans, 64754 Hesseneck-Kailbach (DE)

(57) **Zusammenfassung**

Die Erfindung beschreibt ein analytisches Hilfsmittel (1), das für die Gewinnung und Untersuchung von Körperflüssigkeiten, insbesondere Blut, geeignet ist. Das Hilfsmittel (1) enthält ein Testelement (2) und eine Lanzette (3), wobei das Testelement (2) folgende Komponenten enthält: 1.) ein Rahmenelement (7) und 2.) zumindest ein Nachweiselement (6), welches auch mehrschichtig sein kann und unter anderem eine Erythrozytenabtrennschicht, eine Spreitschicht und eine optische Sperrschicht aufweisen kann, und das direkt oder indirekt mit dem Rahmenelement (7) verbunden ist; und die Lanzette (3) folgende Komponenten enthält: 1.) eine Nadel (11) mit Spitze (23) und 2.) einen Lanzettenkörper (10), der die Nadel (11) zumindest teilweise umgibt. Das Hilfsmittel (1) der Erfindung zeichnet sich dadurch aus, dass der Lanzettenkörper (10) beweglich, z. B. klappbar oder schwenkbar, mit dem Rahmenelement (7) des Testelements (2) verbunden ist, so dass die Lanzette (3) eine Aufbewahrungsposition und eine Stechposition einnehmen kann, wobei die Nadel (11) in der Aufbewahrungsposition im wesentlichen parallel zur Ebene des Testelements (6) und in der Stechposition im wesentlichen orthogonal zur Ebene des Testelements (6) orientiert ist.

## Beschreibung

Die vorliegende Erfindung betrifft analytische Hilfsmittel für die Gewinnung und Untersuchung von Körperflüssigkeiten, insbesondere Blut, enthaltend ein Testelement und eine Lanzette, wobei das Testelement ein Rahmenelement und zumindest ein Nachweiselement, das direkt oder indirekt mit dem Rahmenelement verbunden ist, und die Lanzette eine Nadel mit scharfer Spitze und einen Lanzettenkörper, der die Nadel zumindest teilweise umgibt, enthält. Weiterhin betrifft die Erfindung ein System zur Bevorratung von analytischen Hilfsmitteln sowie ein System zur Bestimmung der Anwesenheit oder des Gehalts eines Analyten in Blut, umfassend ein Messgerät zum Messen und Anzeigen der Änderung einer mit dem Analyten korrelierten, charakteristischen Eigenschaft eines Testelements und ein Magazin, welches zur Aufnahme von analytischen Hilfsmitteln geeignet ist.

Zur qualitativen oder quantitativen analytischen Bestimmung von Bestandteilen von Körperflüssigkeiten, insbesondere von Blut, werden oft sogenannte trägergebundene Tests verwendet. Bei diesen trägergebundenen Tests liegen Reagenzien auf oder in entsprechenden Schichten eines festen Testträgers vor, der mit der Probe in Kontakt gebracht wird. Die Reaktion von flüssiger Probe und Reagenzien führt zu einem nachweisbaren Signal, insbesondere einer Farbänderung oder einer Strom- beziehungsweise Potentialänderung. Das Nachweissignal kann visuell oder mit Hilfe eines Geräts - im Fall einer Farbänderung meistens reflexionsfotometrisch, im Fall der Strom- beziehungsweise Potentialänderung amperometrisch beziehungsweise potentiometrisch - ausgewertet werden.

Testträger sind häufig als Teststreifen ausgebildet, die im wesentlichen aus einer länglichen Tragschicht aus Kunststoffmaterial und darauf angebrachten Nachweisschichten als Testfeldern bestehen. Es sind jedoch auch Testträger bekannt, die als quadratische oder rechteckige Plättchen gestaltet sind oder bei denen die funktionellen Schichten von einem Kunststoffrahmen gehalten werden. Allgemein soll im Folgenden deshalb von "Testelementen" gesprochen werden.

Die Bestimmung des Gehalts an bestimmten Analyten in Blut, beispielsweise von Glucose oder Lactat, erfordert die Gewinnung einer ausreichenden Probenmenge (Blut) sowie die Bereitstellung eines geeigneten Messsystems für den Analyten. Neben Arztpraxen und Analysenlabors führen vermehrt medizinische Laien solche Bestimmungen für den Eigengebrauch durch. Insbesondere für die Bestimmung und Kontrolle des Blutzuckerwertes, das heisst des Blutglukosegehaltes, bei Diabetikern, aber auch für die Ermittlung anderer Parameter wie Laktatgehalt oder Cholesterinspiegel, sind von der zu untersuchenden Person selbst anzuwendende Messsysteme weit verbreitet.

Herkömmliche Messsysteme enthalten oftmals Testelemente in Form sogenannter Teststreifen, die zusammen mit entsprechenden Messgeräten die Bestimmung eines oder mehrerer Analyten in Blut erlauben. Daneben benötigt der Anwender weiterhin im Allgemeinen eine Lanzette, mit deren Hilfe die Haut an bestimmten Körperpartien, beispielsweise an der Fingerbeere oder dem Ohrläppchen, durchstochen und so Blut gewonnen werden kann, das für die Messung verwendet werden soll. Für eine komfortable Blutgewinnung bieten verschiedene Hersteller sogenannte Stechhilfen an, welche die Lanzetten kontrolliert und geführt in die Haut stechen, um somit die Einstichtiefe zu kontrollieren und den Schmerz zu minimieren.

Da der Anwender zur Messung eines Analyten in Blut mehrere separate Komponenten benötigt (Testelemente, Messgerät, Stechhilfe, Lanzetten etc.) und für eine Analyse "außer Haus", beispielsweise auf Reisen oder beim Sport, mit sich führen muss, ist es verständlich, dass vor allem Diabetiker, die zusätzlich auch noch Insulin und eine Spritze mit sich führen müssen, eine Reduzierung der mitzuführenden Einzelteile für wünschenswert erachten.

Deshalb mangelt es nicht an unterschiedlichen Versuchen, die Anzahl der Einzelteile, die für eine Blutparameterbestimmung notwendig sind, zu reduzieren. Eine im Stand der Technik beschriebene Lösung besteht darin, die benötigten Komponenten wie Messgerät, Stechhilfe, Lanzetten und Testelemente in ein gemeinsames Blutgewinnungs- und Messsystem zu vereinigen, wobei insbesondere Lanzette und Testelement zu einem einzigen analytischen Hilfsmittel kombiniert werden. Vorteilhafterweise können diese analytischen Hilfsmittel magaziniert und automatisiert bereitgestellt, benutzt, ausgewertet und entsorgt werden.

Im Stand der Technik lassen sich prinzipiell zwei unterschiedliche Ansätze für analytische Hilfsmittel ausmachen, bei denen ein flächenförmiges Testelement mit einer Lanzette in einem einzigen Gegenstand (analytisches Hilfsmittel) vereint sind: Zum einen sind Konzepte beschrieben, bei denen die Lanzette bzw. deren Nadel oder Dorn eine Stechbewegung im wesentlichen senkrecht zur Ebene des Testelements vollführt (so zum Beispiel beschrieben in US 5,035,704 und den Figuren 2 und 3 von DE-A 198 55 443, DE-A 198 55 458 und DE-A 198 55 465); zum anderen verläuft diese Stechbewegung derart, dass die Lanzette bzw. deren Nadel oder Dorn, sich weitgehend parallel zur Ebene des Testelements bewegt (vgl. hierzu Figur 1 in DE-A 198 55 443, DE-A 198 55 458 und DE-A 198 55 465). Beiden Varianten ist gemeinsam, dass die Orientierung der Lanzette relativ zum Testelement sowohl bei der Ausführung der Stechbewegung (Stechposition) als auch im "Ruhezustand" (Aufbewahrungsposition) gleich bleibt.

Ein in Stapeln magazinierbares Testelement mit integrierter Lanzette ist in US 5,035,704 beschrieben. Das Testelement besteht aus einem flachen, rechteckigen (Kunststoff)-Rahmen, in den auf der Oberseite die Lanzette eingelegt ist und der auf der Unterseite parallel zur Grundfläche des Rahmens ein Nachweiselement trägt. Das Nachweiselement bedeckt nur einen Teil der Unterseite des Rahmens; der verbleibende Teil ist praktisch offen und dient als Durchtrittsöffnung für die Lanzettenspitze. Zwischen Lanzette und Nachweiselement befindet sich ein Dochtmaterial, welches das Nachweiselement vollflächig bedeckt und in den vom Nachweiselement nicht bedeckten Anteil der Grundfläche ragt. Das Dochtmaterial soll Blut von der Einstichstelle der Lanzette in die Haut zum Nachweiselement transportieren. Die Lanzette besteht aus einem mit Ausstanzungen versehenen Metallblech, das auf der Unterseite, das heisst dem Nachweiselement zugewandten Seite, einer zentralen, beweglichen Zunge einen spitzen, im wesentlichen senkrecht zum Metallblech (und damit zur Ebene des Nachweiselements) ausgerichteten Metalldorn trägt. Die Spitze des Dorns liegt im Ruhezustand des Testelements innerhalb der Begrenzungsflächen des Testelementerahmens.

Bei Benutzung des Testelements aus US 5,035,704 wird das Testelement mit der Unterseite auf die Hautoberfläche aufgelegt und die bewegliche Zunge mit dem Dorn von oben mit einem Stössel nach unten bewegt. Der Dorn tritt dabei durch eine Aussparung im Dochtmaterial in die Hautoberfläche ein und erzeugt eine kleine Wunde, aus der nach dem Zurückziehen des Dorns Blut austritt. Das Blut wird vom Dochtmaterial des auf der Hautoberfläche aufliegenden Testelements zum Nachweiselement geleitet und dort analysiert.

DE-A 198 55 443, DE-A 198 55 458 und DE-A 198 55 465 beschreiben unter anderem unterschiedliche Ausführungsformen von analytischen Hilfsmitteln, die ein Testelement und eine Lanzette enthalten und darin als "Testkassette" bezeichnet werden.

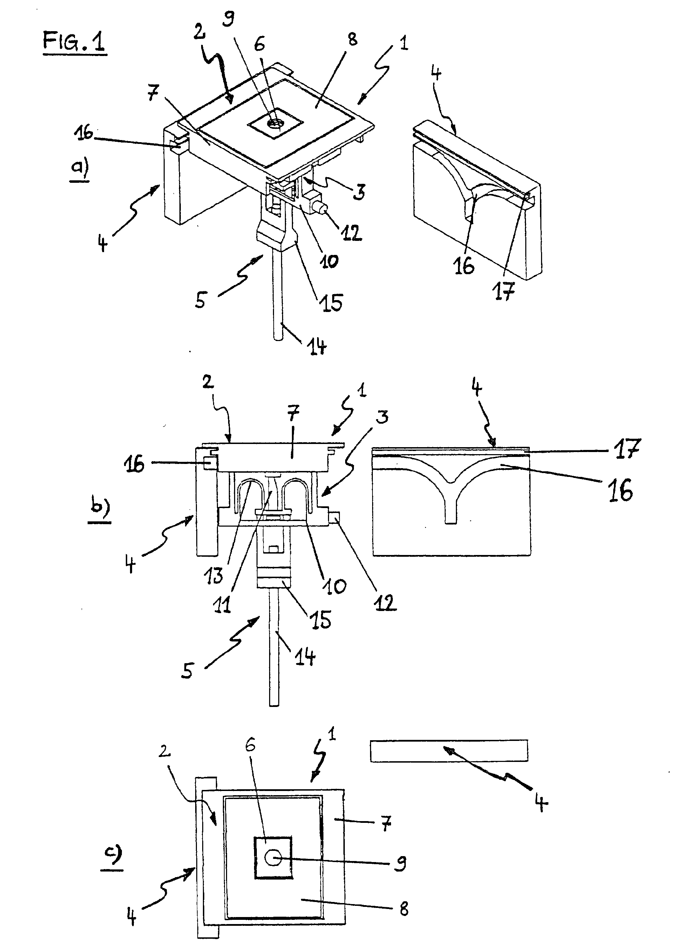

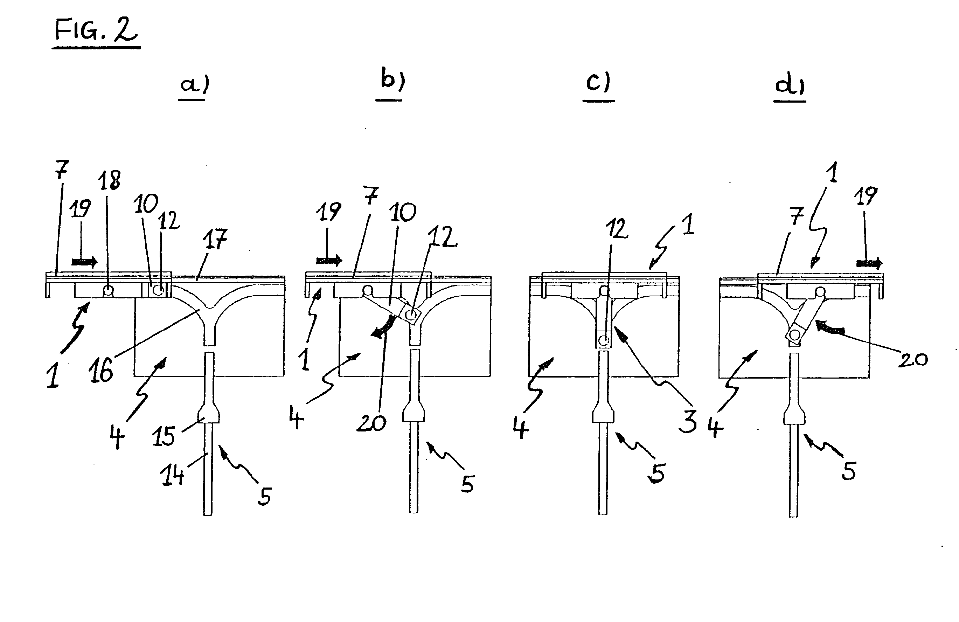

In den Figuren 1 und 2 (und den dazugehörigen Passagen der Erfindungsbeschreibung) der drei genannten Offenlegungsschriften sind flache, im wesentlichen rechteckige "Testkassetten" offenbart, bei denen in einen mehrteiligen Kunststoffrahmen ein Lanzettenelement eingelegt ist. Dieses besteht aus einer Lanzettennadel, die von einem Kunststoffrahmen gehalten wird. Der Kunststoffrahmen erfüllt dabei die Rolle eines Führungselements für die Lanzettennadel und dient zudem dazu, die Lanzettennadel quasi als Federelement nach erfolgter Stechbewegung in die Ausgangsposition zurückzubewegen. Anders als dies bei dem Testelement aus US 5,035,704 der Fall ist, verläuft die Stechbewegung der Lanzettennadel in den in den Figuren 1A bis 1E und 2A bis 2B von DE-A 198 55 458 beschriebenen Ausführungsformen im wesentlichen parallel zur Ebene des Nachweiselements. Dieses kann entweder über einen Kapillarkanal, der entweder im Bereich der Austrittsöffnung der Lanzettennadel beginnt oder an einer beliebigen Stelle des Testkassettengehäuses liegt, mit Blut versorgt werden.

Figur 2C von DE-A 198 55 458 beschreibt eine ähnliche Testkassette, bei der das Nachweiselement in einer Seitenfläche des Kunststoffrahmens liegt und die Austrittsöffnung der Lanzettennadel umgibt. In dieser Ausführungsform verläuft die Stechbewegung der Lanzettennadel im wesentlichen orthogonal zur Ebene des Nachweiselements.

Figur 3 von DE-A 198 55 443, DE-A 198 55 458 und DE-A 198 55 465 offenbart längliche, zylindrische "Testkassetten", in denen die Lanzette in einer Kunststoffhülse geführt wird und die Lanzettennadel beim Ausführen der Stechbewegung aus der Grundfläche der "Testkassette" austritt. Die Austrittsöffnung der Lanzettennadel kann von einem Nachweiselement umgeben sein. Wie dies auch bei dem Testelement aus US 5,035,704 der Fall ist, verläuft die Stechbewegung der Lanzettennadel in der in Figur 3 von DE-A 198 55 443, DE-A 198 55 458 und DE-A 198 55 465 beschriebenen Ausführungsform im wesentlichen senkrecht zur Ebene des Nachweiselements. Die Lanzettennadel ist in dieser Ausführungsform von einem Lanzettenkörper umgeben, der im Zusammenspiel mit der zylindrischen Kunststoffhülse der "Testkassette" die Führung der Lanzettennadel beim Stechvorgang bewirkt. Lanzettenkörper und zylindrische Kunststoffhülse wirken weiterhin mit einer Schraubenfeder zusammen, so dass nach erfolgter Stechbewegung die Lanzettennadel in die Ausgangsposition zurückbewegt wird.

Die Aufgabe der vorliegenden Erfindung ist es, ein analytisches Hilfsmittel enthaltend ein Testelement und eine Lanzette bereitzustellen, welches leicht und sicher stapelbar, und bezüglich der Lanzette so aufbewahrbar ist, dass ein unbeabsichtigtes Verletzen an der Lanzette weitgehend ausgeschlossen ist. Zusätzlich soll das analytische Hilfsmittel sich durch kleine Dimensionen auszeichnen und für eine automatisierte Handhabung in einem Analysengerät geeignet sein.

Die Aufgabe wird durch den Gegenstand der Erfindung, wie er in den Patentansprüchen definiert ist, gelöst.

Gegenstand der Erfindung ist ein analytisches Hilfsmittel, das sowohl für die Gewinnung als auch für die unmittelbar anschließende Untersuchung von Körperflüssigkeiten, insbesondere Blut, geeignet ist. Das erfindungsgemäße analytische Hilfsmittel enthält ein Testelement und eine Lanzette. Das Testelement umfasst ein Rahmenelement, zumindest ein Nachweiselement, das direkt oder indirekt mit dem Rahmenelement verbunden ist, und gegebenenfalls weitere Komponenten, wie zum Beispiel eine Zone, die zur Aufnahme überschüssiger Probenflüssigkeit geeignet ist, und einen Träger für das Nachweiselement und/oder die Zone, die zur Aufnahme überschüssiger Probenflüssigkeit geeignet ist. Die Lanzette enthält eine Nadel mit einer Spitze und einen Lanzettenkörper, der die Nadel zumindest teilweise umgibt. Wesentlich für das erfindungsgemäße analytische Hilfsmittel ist, dass der Lanzettenkörper beweglich, das heisst klappbar oder verschwenkbar, mit dem Rahmenelement des Testelements verbunden ist, so dass die Lanzette eine Aufbewahrungsposition und eine von der Aufbewahrungsposition verschiedene Stechposition einnehmen kann. Die Nadel liegt in der Aufbewahrungsposition im wesentlichen parallel zur Ebene des Testelements. In der Stechposition hingegen ist sie im wesentlichen orthogonal zur Ebene des Testelements orientiert. Die Spitze der Nadel weist in der Stechposition im wesentlichen in Richtung des Testelements.

Als "analytisches Hilfsmittel" im erfindungsgemäßen Sinn wird eine Vorrichtung verstanden, die zumindest aus einem Testelement mit integrierter Lanzette besteht. Die Lanzette dient dabei der Gewinnung einer Körperflüssigkeitsprobe, welche anschließend mit Hilfe des Testelements auf die Anwesenheit und/oder den Gehalt eines Analyten hin untersucht wird. Das erfindungsgemäße analytische Hilfsmittel ist also zur Gewinnung einer Körperflüssigkeit, insbesondere von Blut, einer zu untersuchenden Person geeignet. Dabei durchstößt die Lanzette kurzzeitig und mit definierter Einstichtiefe die Haut dieser Person, wodurch eine winzige Wunde entsteht. Auf der Oberfläche der Wunde sammelt sich ein Tropfen der Körperflüssigkeit, insbesondere Blut, von in aller Regel weniger als einem Mikroliter bis maximal 100 Mikroliter Volumen. Vorzugsweise wird die Körperflüssigkeit direkt im Anschluss an die Gewinnung für eine diagnostische Untersuchung mit Hilfe des Testelements eingesetzt.

Insbesondere kann mit dem erfindungsgemäßen analytischen Hilfsmittel Blut, vorzugsweise Kapillarblut, aus einer Körperpartie, wie z. B. einer Fingerbeere oder einem Ohrläppchen, eines Individuums gewonnen werden. Das analytische Hilfsmittel kann sowohl von dem zu untersuchenden Individuum selbst, beispielsweise einem Diabetiker, der seinen Blutglukosegehalt bestimmen möchte, als auch von einem Dritten, z. B. einem Arzt oder einer Krankenschwester, zur Gewinnung und Untersuchung von Blutproben, angewendet werden.

Vorzugsweise ist das analytische Hilfsmittel zur Aufbewahrung in einem Magazin geeignet und kann in einem weitgehend automatisierten Prozess in einem Messgerät ausgewertet werden. Beispielsweise können erfindungsgemäße analytische Hilfsmittel übereinanderliegend als Stapel oder nebeneinanderliegend in Form einer Kette magaziniert werden.

Das Testelement enthält eine formgebende, steife Komponente, die im folgenden als Rahmenelement bezeichnet werden soll. Das Rahmenelement dient der mechanischen Stabilisierung des Nachweiselements, der einfachen Handhabbarkeit des analytischen Hilfsmittels und sorgt im Zusammenspiel mit der klappbaren Lanzette dafür, dass die Gefahr einer unbeabsichtigten Verletzung an der Lanzettennadel vermieden wird.

Das Rahmenelement kann in einer besonders einfachen Ausführungsform des erfindungsgemäßen Testelements eine steife, beispielsweise rechteckige Kunststofffolie oder ein steifer Kartonstreifen sein, der auf einer Seite das Nachweiselement trägt und auf der anderen Seite beweglich mit der Lanzette verbunden ist, beispielsweise über ein Filmscharnier oder einen Klebebandstreifen. Damit die Lanzettennadel beim Stechvorgang gegebenenfalls durch das Rahmenelement hindurchdringen kann, kann es eine Aussparung aufweisen.

Vorzugsweise ist das Rahmenelement jedoch ein im wesentlichen rechteckiges, flaches Kunststoffformteil, beispielsweise ein Spritzgussteil. Das Rahmenelement kann neben dem eigentlichen Rahmen in seinem Inneren eine Auflagefläche für das Nachweiselement bereitstellen. Es ist jedoch auch möglich, dass das Nachweiselement nur an seinen Kanten vom Rahmen gehalten wird. In beiden Fällen ist das Nachweiselement fest mit dem Rahmenelement verbunden.

Selbstverständlich kann das Rahmenelement auch mehrteilig sein. Beispielsweise kann das Rahmenelement aus zwei Hälften bestehen und das Nachweiselement - ähnlich dem Rähmchen eines Diapositivs - zwischen die beiden Hälften eingeklemmt halten.

Entsprechende Testelemente sind aus dem Stand der Technik bekannt, beispielsweise aus EP-A 0 885 591, EP-B 0 535 480 und EP-B 0 477 322.

Alternativ dazu kann das Nachweiselement auf einer vom Rahmenelement verschiedenen Trägerschicht, beispielsweise einer transparenten Kunststofffolie, befestigt sein, welche wiederum vom Rahmenelement gehalten wird. Diese Variante kann Vorteile bei der Fertigung des analytischen Hilfsmittels aufweisen. In diesem Fall ist das Nachweiselement indirekt-über die Trägerschicht - mit dem Rahmenelement verbunden.

Das Nachweiselement besteht im wesentlichen aus einer reagenzienhaltigen Nachweisschicht, die auf einem Träger, beispielsweise der Auflagefläche des oben beschriebenen Rahmenelements oder einer separaten Trägerschicht, befestigt ist. Die Nachweisschicht enthält die Reagenzien, die zum Nachweis des Zielanalyten in der zu untersuchenden Probenflüssigkeit erforderlich sind. Die Reagenzien in der Nachweisschicht führen bei Anwesenheit des Zielanalyten in der Probenflüssigkeit zu einem direkt oder indirekt optisch oder elektrisch beobachtbaren Signal, vorzugsweise einer Farbänderung oder einem Stromfluss. Die Nachweisschicht kann beispielsweise in Form eines mit analytischen Reagenzien und Hilfsstoffen getränkten Papiers oder einer mit Reagenzien, Füllstoffen und Filmbildnern beschichteten Kunststoffolie ausgebildet sein. Es ist ebenfalls möglich, dass das Nachweiselement eine reagenzienhaltige Membran oder eine reagenzienbeschichtete Elektrode ist. Solche Nachweiselemente, die erfindungsgemäß für das analytische Testelement geeignet sind, sind dem Fachmann in einer Vielzahl von Ausführungsformen bekannt, beispielsweise aus EP-A 0 821 234, EP-B 0 575 364, EP-A 0 016 387, EP-A 750 196, EP-A 0 654 659.

Als analytische Reagenzien sind im Zusammenhang mit der vorliegenden Erfindung prinzipiell jegliche Art von Nachweisreagenzien und sonstigen reaktiven Hilfsstoffen, wie sie üblicherweise in analytischen und/oder diagnostische Testelementen Verwendung finden, gemeint. Ohne abschließend sein zu wollen sind dies unter anderem Indikatoren, Mediatoren, Markierungsstoffe, Puffersubstanzen, Spreit- und Netzmittel, Aktivatoren, biochemische Reagenzien, Enzyme, Proteine, Peptide, Antigene oder Antikörper und deren Fragmente, Haptene, und/oder Nucleinsäuren. Solche Reagenzien sind dem Fachmann für eine Vielzahl analytischer und/oder diagnostischer Fragestellungen bekannt. Auch falls im folgenden Text oftmals von "Reagenzien" gesprochen wird, soll auch die Möglichkeit, dass nur ein Reagenz eingesetzt wird, erfindungsgemäß miteingeschlossen sein.

Erfindungsgemäß geeignete Nachweiselemente müssen nicht zwingend aus nur einer einzigen Schicht bestehen. Es ist vielmehr möglich, dass das Nachweiselement aus zwei oder mehreren Schichten unterschiedlicher Funktion, die horizontal oder vertikal nebeneinander angeordnet sein können, besteht. Beispielsweise kann das Nachweiselement mehrschichtig sein und unter anderem eine Erythrozytenabtrennschicht, eine Spreitschicht und eine optische Sperrschicht enthalten.

In einer bevorzugten Ausführungsform kann das Testelement mehrere Nachweiselemente enthalten. Diese können beispielsweise dazu dienen, einen Analyten in unterschiedlichen Konzentrationen zu bestimmen oder für unterschiedliche Analyten spezifisch sein.

Weiterhin kann eine Nachweisschicht für einen oder mehrere Zielanalyten spezifisch sein. Bei Spezifität für mehrere Analyten können die Reagenzien für die unterschiedlichen Analyten in getrennten Bereichen der Nachweisschicht untergebracht sein, so dass eine eindeutige Zuordnung von Analyt und Nachweisergebnis möglich ist. Techniken für die Herstellung solcher Nachweisschichten mit mehreren, getrennten Bereichen sind dem Fachmann bekannt. Beispielsweise seien genannt Druckverfahren wie Siebdruck, Tintenstrahldruck, photolithographische Methoden, oder einfach das Befestigen unterschiedlich imprägnierter Testpapiere auf einem gemeinsamen Träger.

Das Testelement kann neben dem Nachweiselement weitere Komponenten enthalten. Beispielsweise kann eine Zone zur Aufnahme überschüssiger Probenflüssigkeit im Testelement vorgesehen sein. Eine solche Zone liegt vorzugsweise in direkter Nachbarschaft zum Nachweiselement. Sie kann mit dem Nachweiselement in einem flüssigkeitsübertritt-ermöglichendem Kontakt stehen, beispielsweise durch Stoß-an-Stoß-Lage, durch geringfügiges Überlappen an den Berührungskanten oder durch einen Verbindungskanal, so dass überschüssige Probenflüssigkeit direkt in die Zone fliessen kann.

In einer bevorzugten Ausführungsform kann das Testelement eine Trägerschicht für das Nachweiselement und gegebenenfalls weitere Komponenten enthalten. Die Trägerschicht ist in dieser Ausführungsform vorzugsweise direkt mit dem Rahmenelement verbunden, so dass das Nachweiselement wiederum indirekt mit dem Rahmenelement verbunden ist.

Als Trägerschicht eignen sich z. B. Kunststoffolien und -formteile, beschichtete Pappen, Glas, Keramik, Metallbleche und dergleichen mehr. Vorzugsweise sollte die Trägerschicht inert gegenüber den verwendeten Probenmaterialien und Reagenzien sein, nicht von diesen angegriffen werden oder mit ihnen reagieren. Als erfindungsgemäß geeignet haben sich beispielsweise Folien aus inerten, wasserbeständigen Kunststoffen wie Polyethylen, Polypropylen, Polystyrol, Polycarbonat, Polyethylenterephthalat, Polyamid und dergleichen mehr erwiesen.

Für den bevorzugten Fall, dass optisch auszuwertende Testelemente eingesetzt werden sollen, ist dafür zu sorgen, dass die Trägerschicht bzw. die Auflagefläche des Rahmenelements für optische Messverfahren durchlässig sind, falls die Messung von der Seite des Testelements aus durchgeführt werden soll, die auf der Trägerschicht bzw. der Auflagefläche des Rahmenelements aufliegt. Trägerschicht und/oder Auflagefläche können beispielsweise aus einem transparenten Material bestehen oder eine Öffnung besitzen, die eine optische Messung erlaubt. Solche Maßnahmen sind an sich dem Fachmann geläufig.

Die erfindungsgemäß im analytischen Hilfsmittel enthaltene Lanzette besitzt eine Nadel aus Metall, Keramik oder Kunststoff, deren eines Ende (die Spitze) spitz ausgeformt ist, und gegebenenfalls - beispielsweise durch einen Schleifprozess - scharf geschliffen ist. Zumindest der hintere, von dieser Spitze abgewandte Teil der Lanzettennadel (das stumpfe Ende) ist von einem Lanzettenkörper aus Kunststoff ganz oder teilweise umschlossen. Die Herstellung erfolgt üblicherweise derart, dass die Lanzettennadel in einer Kunststoffspritzform positioniert und der Lanzettenkörper angespritzt wird. Es ist auch möglich, dass der Lanzettenkörper aus mehreren, miteinander verbundenen Teilen besteht.

Der Lanzettenkörper dient der Halterung und Führung der Lanzettennadel und stellt die Verbindung zwischen Lanzette und Testelement her. Erfindungswesentlich ist, dass der Lanzettenkörper, beispielsweise über ein Scharnier oder Gelenk, beweglich, das heisst klappbar oder verschwenkbar, mit dem Rahmenelement des Testelements verbunden ist. Der Lanzettenkörper und der Rahmen des Testelements können dabei in einer bevorzugten Ausführungsform einstückig als Spritzgussformteil gefertigt werden. Die Verbindung von Lanzettenkörper und Rahmenelement erfolgt in diesem Fall vorzugsweise über ein Filmscharnier. Alternativ können Rahmenelement und Lanzettenkörper einzelne Spritzgussteile sein, die über ein Gelenk oder Scharnier miteinander beweglich verbunden sind.

Auf diese Weise kann die Lanzette zumindest zwei definierte Orientierungen relativ zur Testelementebene bzw. zur Ebene des Testelements einnehmen: Zum einen kann die Lanzettennadel (und damit auch die Lanzette) parallel zur Ebene des Testelements liegen, was im Folgenden als "Aufbewahrungsposition" bezeichnet werden soll; zum anderen ist eine Orientierung möglich, bei der die Lanzettennadel im wesentlichen orthogonal, das heisst rechtwinklig, zur Ebene des Testelements liegt, wobei die Spitze der Nadel in Richtung des Testelements zeigt. Die letztgenannte Lage der Lanzette soll als "Stechposition" bezeichnet werden. Bevorzugt ist weiterhin, dass das Nachweiselement im wesentlichen parallel zum Testelement liegt.

In der Aufbewahrungsposition ist ein platzsparendes Stapeln der erfindungsgemäßen analytischen Hilfsmittel möglich. Die parallele Ausrichtung der Lanzette relativ zum Testelement führt zu kompakten Außenmaßen des analytischen Hilfsmittels. Vorzugsweise kann die Lanzette annähernd vollständig innerhalb der Begrenzungsflächen im Rahmenelement des Testelements verstaut werden, wie anhand der nachfolgenden Figuren noch näher beschrieben werden wird. Auf diese Weise wird erreicht, dass die Spitze der Lanzettennadel nicht freiliegt und so das Verletzungsrisiko minimiert wird. Außerdem wird ein einfaches Stapeln mehrerer analytischer Hilfsmittel möglich.

In der Stechposition ist die Lanzette aus der Ebene des Testelements ausgeklappt und steht senkrecht zu ihr. Vorzugsweise ist die Nadelspitze der Lanzettennadel auch in dieser Position zunächst innerhalb der Begrenzungsflächen des Rahmenelements verborgen, so dass ein unbeabsichtigtes Verletzen an der Nadelspitze nahezu ausgeschlossen ist. Das Ende der Lanzette, das mit dem Lanzettenkörper umgeben ist, ragt in der Stechposition aus den Begrenzungsflächen des Rahmenelements heraus und kann in dieser exponierten Position leicht von einem Antriebselement erfasst werden.

Die Stechbewegung der Lanzette wird durch Einwirken eines Antriebselements, beispielsweise eines Hammers oder Stössels, der Teil eines entsprechenden Messgeräts bzw. einer entsprechenden Stechhilfe ist, auf das stumpfe Ende der Lanzettennadel bzw. den am stumpfen Ende der Lanzettennadel befindlichen Lanzettenkörper initiiert. Das Antriebselement kann dabei sowohl die Vorwärtsbewegung der Lanzettennadel, das heisst die Bewegung in Richtung der zu stechenden Hautpartie, als auch deren Rückwärtsbewegung in die Ausgangslage bewirken. Vorzugsweise wirkt das Antriebselement linear auf die Lanzette ein, die ihrerseits dann eine lineare Stechbewegung vollführt.

Wie nachfolgend im Zusammenhang mit den Figuren noch näher beschreiben wird bewirkt in einer bevorzugten Ausführungsform das Antriebselement zunächst ein Vorspannen der Rückholfeder der Lanzette. Erst in einem nachfolgenden Schritt wird die Lanzettennadel vorwärts, das heisst in Richtung der zu stechenden Hautoberfläche, getrieben. Durch das Vorspannen der Rückholfeder wird erreicht, dass die Stechbewegung der Nadel vollständig vom Antriebselement des Messgeräts bzw. der Stechhilfe gesteuert wird. Auf diese Weise wird ein gesteuerter, kontrollierter Weg-Zeit-Verlauf für die Lanzettennadel während der Stechbewegung erreicht, der zu einem möglichst schmerzarmen Stechen führt.

Die Lanzette durchdringt beim Stechen vorzugsweise die Ebene des Nachweiselements. Das bedeutet jedoch nicht zwangsläufig, dass die Lanzettennadel durch das Nachweiselement dringen muss, obwohl dies - wenn auch weniger bevorzugt - möglich ist. In einer bevorzugten Ausführungsform enthält das Testelement im Bereich des Nachweiselements eine Aussparung oder Durchtrittsöffnung, durch die die Lanzettennadel beim Stechvorgang ohne das Nachweiselement zu berühren hindurchtreten kann. Vorzugsweise umgibt das Nachweiselement diese Durchtrittsöffnung. Alternativ kann eine solche Aussparung auch in einem anderen Bereich des Testelements liegen, beispielsweise im Bereich des Rahmenelements oder im Bereich anderer funktionaler Zonen des Testelements. Es ist auch möglich, dass die Lanzettennadel bei ihrer Stechbewegung außen am Rahmenelement des Testelements vorbei geführt wird.

Vorzugsweise liegt das Nachweiselement in unmittelbarer Nachbarschaft zur Durchtrittsöffnung der Lanzettennadel. Auf diese Weise wird gewährleistet, dass der Benutzer des erfindungsgemäßen analytischen Hilfsmittels nach dem Stechen die gestochene Körperpartie, beispielsweise den gestochenen Finger, zur Aufgabe der Blutprobe auf das Testelement nicht in eine andere Position bringen muss. Vielmehr kann die Körperpartie zu diesem Zweck an der selben Stelle verbleiben, so dass die sich auf der Haut sammelnde Blutprobe quasi von selbst auf das Nachweiselement aufgegeben wird.

Alternativ dazu kann im Bereich der Durchtrittsöffnung der Beginn einer kapillaren Transportstrecke vorgesehen sein, mit deren Hilfe eine Blutprobe auf ein sich nicht in unmittelbarer Nachbarschaft zur Durchtrittsöffnung befindliches Nachweiselement transportiert werden kann. Beipielsweise kann die Transportstrecke ein Kapillarkanal oder ein saugfähiger Docht sein, die jeweils in einem flüssigkeitsübertrittermöglichendem Kontakt mit dem oder den Nachweiselement(en) stehen.

Um zu vermeiden, dass Blut, das beim Stechen durch die Lanzette gewonnen wird und sich als Tropfen auf der Hautoberfläche sammelt, während der Benutzung des analytischen Hilfsmittels durch die Durchtrittsöffnung auf die der Haut abgewandten Seite des Testelements gelangt, kann die Durchtrittsöffnung mit einer Membran aus einem elastischen Kunststoff verschlossen sein. Die Membran wird - ähnlich einem Septum - beim Stechvorgang von der Lanzettennadel durchdrungen und schließt sich aufgrund ihrer Elastizität nach dem Zurückziehen der Nadel wieder.

Rahmenelement und Lanzettenkörper sind vorzugsweise Spritzgussteile aus einem spritzgussfähigen Material, insbesondere einem zum Spritzgiessen geeigneten Kunststoff. Solche Kunststoffe sind dem Fachmann bekannt, z. B. Polystyrol, Polyamide, Polyurethane, Celluloseether und -ester, Polyethylen, Polymethacrylsäureester und andere Thermoplaste, aushärtende Duroplaste bzw. vulkanisierende Elastomere aus Kautschuk oder Siliconkautschuk und - wenn auch weniger bevorzugt - Schaumkunststoffe. In einer bevorzugten Ausführungsform sind das Rahmenelement und der Lanzettenkörper einteilig gefertigt und über ein Filmscharnier miteinander verbunden.

Um die Lanzettennadel nach erfolgter Stechbewegung in ihre Ausgangsposition zurückzuholen, kann der Lanzettenkörper eine Rückholfeder enthalten. Diese ist vorzugsweise direkt mit der Lanzettennadel oder dem die Lanzettennadel umgebenden Teil des Lanzettenkörpers verbunden. Die Rückholfeder kann beispielsweise eine Blattfeder oder eine Schraubenfeder aus Metall sein, die beim Stechvorgang zusammengedrückt wird und deren Zurückfedern in den entspannten Zustand die Lanzettennadel in ihre Ausgangsposition bewegt. Vorzugsweise ist die Rückholfeder jedoch ein deformierbarer, elastischer Teil des Lanzettenkörpers, beispielsweise aus dem selben Kunststoff einstückig spritzgegossen wie der übrige Teil des Lanzettenkörpers. Natürlich ist es auch möglich, für die Rückholfeder einen anderen Kunststoff zu verwenden als für den übrigen Lanzettenkörper. Dies ist beispielsweise in Zweikomponentenspritzgussfertigungsprozessen möglich.

Um die Stechbewegung der Lanzettennadel zu optimieren kann in einer bevorzugten Ausführungsform des erfindungsgemäßen analytischen Hilfsmittels im Lanzettenkörper und/oder im Testelement, insbesondere im Rahmenelement des Testelements, ein Führungselement, beispielsweise in Form einer Führungshülse oder eines Führungskanals im Testelement oder in Form einer doppelten Blattfeder im Lanzettenkörper, vorhanden sein.

Für den Einsatz in weitgehend automatisierten Testsystemen hat es sich als vorteilhaft herausgestellt, dass das Testelement, insbesondere das Rahmenelement des Testelements, an zwei gegenüberliegenden Außenseiten ein Führungsprofil besitzt, welches in ein komplementäres Profil in den entsprechenden Teilen eines Magazins oder eines Testgerätes eingreift. Auf diese Weise kann die Bewegung des analytischen Hilfsmittels geführt werden und auch seine Fixierung in der Messposition ist möglich. Beispielsweise kann das erfindungsgemäße analytische Hilfsmittel von einer Vorratsposition, zum Beispiel in einem Hilfsmittelmagazin, in die Stech- und/oder Messposition in einem Messgerät gebracht werden.

Beim Transport des analytischen Hilfsmittels von der Vorratsposition in die Stech- bzw. Messposition wird - vorzugsweise gleichzeitig - die Lanzette von der Aufbewahrungsposition in die Stechposition gebracht. Dies kann automatisch, ohne äussere Einwirkung geschehen, beispielsweise durch Ausnutzen des Schwerkraft und dadurch bewirktes "Herunterklappen" und Aufrichten der Lanzette. Es ist jedoch bevorzugt, dass die Bewegung der Lanzette von der Aufbewahrungs- in die Stechposition geführt abläuft. Zu diesem Zweck hat es sich als vorteilhaft herausgestellt, dass der Lanzettenkörper zumindest eine, vorzugsweise jedoch ein Paar gegenüberliegender, außenliegender Nocken aufweist, die in entsprechende Führungsnute im Messgerät und/oder dem Magazin eingreifen können. Bevorzugt wird die lineare Bewegung des analytischen Hilfsmittels von der Aufbewahrungsposition in die Messposition durch gekrümmte Führungsnuten für die Nocken des Lanzettenkörpers in ein geeignetes "Herunterklappen" der Lanzette umgesetzt.

In analoger Weise führt ein Weitertransport des analytischen Hilfsmittels nach durchgeführter analytischer Bestimmung von der Stech- bzw. Messposition in die nachfolgende Position (zwecks Auswurf oder Wiedermagazinierung) dazu, dass die Lanzette wieder in die Aufbewahrungsposition zurückgeführt wird. Es ist dabei möglich, dass die Aufbewahrungsposition vor dem Erreichen der Stech- bzw. Messposition und die Aufbewahrungsposition danach identisch oder auch unterschiedlich sind. Wesentlich ist nur, dass die Lanzette aus einer Orientierung orthogonal zur Ebene des Testelements in eine Orientierung parallel zur Ebene des Testelements zurückkehrt. Auf diese Weise wird weitgehend vermieden, dass sich eine Person unbeabsichtigt an der Lanzette des analytischen Hilfsmittels verletzt.

Ein weiterer Gegenstand der Erfindung ist ein System zur Bevorratung von analytischen Hilfsmitteln. Dieses System umfasst ein Magazin oder Vorratsbehältnis für analytische Hilfsmittel und einen Vorrat an den erfindungsgemäßen analytischen Hilfsmitteln. Die analytischen Hilfsmittel können dabei im wesentlichen übereinander in Form eines Stapels oder im wesentlichen hinter- bzw. nebeneinander in Form einer Kette im Magazin aufbewahrt werden.

Vorzugsweise enthält das Magazin komplementäre Führungsprofile für das Rahmenelement der bevorzugten Ausführungsform des analytischen Hilfsmittels. Dadurch kann der sichere und geführte Transport der Hilfsmittel aus dem Magazin gewährleistet werden.

In einer alternativen, ebenfalls bevorzugten Ausführungsform kann das Magazin des erfindungsgemäßen Systems Führungsnuten für die Nocken des Lanzettenkörpers des analytischen Hilfsmittels, die in einer oben beschriebenen, bevorzugten Ausführungsform des analytischen Hilfsmittels enthalten sind, aufweisen. Wie oben beschrieben, wirken die Führungsnuten mit den Nocken zusammen, um beim Transport des Hilfsmittels in die Mess- bzw. Stechposition die Lanzette aus der Aufbewahrungs- in die Stechposition zu bewegen.

Ein weiterer Gegenstand der Erfindung ist ein System zur Bestimmung der Anwesenheit oder des Gehalts eines Analyten in Blut. Das erfindungsgemäße System umfasst ein Messgerät zum Messen und Anzeigen der Änderung einer mit dem Analyten korrelierten, charakteristischen Eigenschaft eines Testelements und ein entsprechendes, in dem Messgerät einsetzbares, oben beschriebenes analytisches Hilfsmittel. Weiterhin kann das System ein Magazin enthalten, das zur Aufnahme der erfindungsgemäßen analytischen Hilfsmittel geeignet ist.

Vorzugsweise enthält das Messgerät komplementäre Führungsprofile für das Rahmenelement der bevorzugten Ausführungsform des analytischen Hilfsmittels. Dadurch kann der sichere und geführte Transport der Hilfsmittel in die Mess- und Stechposition und die Fixierung des analytischen Hilfsmittels in dieser Position gewährleistet werden.

In einer alternativen, ebenfalls bevorzugten Ausführungsform kann das Messgerät des erfindungsgemäßen Systems Führungsnuten für die Nocken des Lanzettenkörpers des analytischen Hilfsmittels, die in einer oben beschriebenen, bevorzugten Ausführungsform des analytischen Hilfsmittels enthalten sind, aufweisen. Wie oben beschrieben, wirken die Führungsnuten mit den Nocken zusammen, um beim Transport des Hilfsmittels in die Mess- bzw. Stechposition die Lanzette aus der Aufbewahrungs- in die Stechposition zu bewegen.

Das erfindungsgemäße System kann vorzugsweise im Messgerät oder im Magazin eine Schub- oder Zugvorrichtung, beispielsweise einen motor- oder federgetriebenen Stössel, für die analytischen Hilfsmittel aufweisen, mit der ein analytisches Hilfsmittel in die Mess- bzw. Stechposition bewegt wird. Weiterhin kann es bevorzugt sein, dass das Messgerät eine Vorrichtung zum Antrieb der Lanzette des erfindungsgemäßen analytischen Hilfsmittels umfasst, vorzugsweise einen Stössel zum Antrieb der Lanzettennadel.

Der erfindungsgemäße Vorteil des sicheren Aufbewahrens der Lanzettennadel in einer Aufbewahrungsposition und des exponierten Darbietens der Lanzettennadel in einer von der Aufbewahrungsposition verschiedenen Stechposition kann natürlich auch von einem Gegenstand genutzt werden, der kein Nachweiselement enthält. Im weitesten Sinne kann ein solcher Gegenstand als klappbare Lanzette bezeichnet werden. Sie ist ein weiterer Gegenstand der Erfindung.

Die Klapplanzette unterscheidet sich vom oben beschriebenen analytischen Hilfsmittel dadurch, daß sie ― wie gesagt ― kein Nachweiselement und somit kein vollständiges Testelement im erfindungsgemäßen Sinne enthält. Sie enthält jedoch das erfindungsgemäße Rahmenelement, mit dem es beweglich, insbesondere klappbar verbunden ist. Das Rahmenelement und die Verbindung der Lanzette mit dem Rahmenelement sowie die Lanzette an sich sind analog dem erfindungsgemäßen Hilfsmittel (enthaltend Testelement und Lanzette) gestaltet. Insbesondere kann die Lanzette der Klapplanzette eine Aufbewahrungsposition und eine davon verschiedene Stechposition einnehmen, wobei sie in der Aufbewahrungsposition im wesentlichen parallel zur Ebene des Rahmenelements, in der Stechposition hingegen im wesentlichen senkrecht zur Ebene des Rahmenelements orientiert ist.

Ein entsprechender weiterer Gegenstand der vorliegenden Erfindung ist ein System zur Bevorratung der erfindungsgemäßen klappbaren Lanzette. Dieses System enthält ein Magazin oder Vorratsbehältnis für klappbare Lanzetten und entspricht ansonsten dem oben dem oben in Zusammenhang mit dem Bevorratungssystem für erfindungsgemäße analytische Hilfsmittel gesagten.

Die Erfindung wird durch die nachfolgenden Figuren näher erläutert.

Figur 1 zeigt in perspektivischer Aufsicht (Fig. 1 a), in einer Frontalansicht (Fig. 1 b) bzw. in einer Aufsicht (Fig. 1 c) eine bevorzugte Ausführungsform des erfindungsgemäßen analytischen Hilfsmittels.

Figur 2 zeigt in einer Abfolge von vier Teilfiguren (Fig. 2 a bis d) schematisch in Seitenansicht das Zusammenwirken der bevorzugten Ausführungsform des analytischen Hilfsmittels aus Figur 1 mit einem Teil eines Messgeräts während des Transports des analytischen Hilfsmittels in die bzw. aus der Messposition.

Figur 3 zeigt in einer Abfolge von vier Teilfiguren (Fig. 3 a bis d) schematisch in Schnittansicht durch die bevorzugte Ausführungsform des analytischen Hilfsmittels aus Figur 1 das Zusammenwirken zwischen analytischem Hilfsmittel und Messgerät während des Stechvorgangs.

Figur 4 ist eine schematische Schnittansicht einer bevorzugten Ausführungsform des erfindungsgemäßen Systems enthaltend ein Messgerät, ein Magazin für analytische Hilfsmittel und die erfindungsgemäß bevorzugte Ausführungsform des analytischen Hilfsmittels aus Figur 1.

Figur 5 zeigt eine schematische perspektivische Aufsicht auf die Unterseite einer bevorzugten Ausführungsform des erfindungsgemäßen analytischen Hilfsmittels. Das analytische Hilfsmittel entspricht dabei im wesentlichen dem in Figur 1 gezeigten.

In den Figuren 6 bis 9 sind weitere alternative bevorzugte Ausführungsformen des erfindungsgemäßen analytischen Hilfsmittels in perspektivischer Aufsicht auf die Unterseite gezeigt.

Figur 10 zeigt in einer Abfolge von 3 Teilfiguren (Figuren 10 a bis c) schematisch in perspektivischer Aufsicht auf die Unterseite des erfindungsgemäßen Testelements (oben) bzw. in schematischer Seitenansicht (unten) das Zusammenwirken einer bevorzugten Ausführungsform des analytischen Hilfsmittels aus Figur 5 mit einem Teil eines Messgeräts während des Transports des analytischen Hilfsmittels in die bzw. aus der Messposition.

Figur 11 zeigt in einer schematischen perspektivischen Aufsicht auf die Unterseite einen Stapel von erfindungsgemäß bevorzugten analytischen Hilfsmitteln, wie sie beispielsweise in dem Magazin gemäß Figur 4 vorliegen.

Die Ziffern in den Figuren bedeuten:
- 1: analytisches Hilfsmittel
- 2: Testelement
- 3: Lanzette
- 4: Führungsplatte
- 5: Lanzettenstössel
- 6: Nachweiselement
- 7: Rahmenelement
- 8: Zone zur Aufnahme überschüssiger Probenflüssigkeit (Waste-Zone)
- 9: Durchtrittsöffnung für Lanzettennadel
- 10: Lanzettenkörper
- 11: Lanzettennadel
- 12: Nocken
- 13: Rückholfeder
- 14: Nadelstössel
- 15: Federstössel
- 16: Nockenführungsnut
- 17: Führungsnut für Rahmenelement
- 18: Scharnier
- 19: Transportrichtung des analytischen Hilfsmittels
- 20: Klapprichtung der Lanzette
- 21: Septum
- 22: Durchtrittsöffnung für Lanzettenstössel
- 23: Nadelspitze der Lanzettennadel
- 24: Führungshülse
- 25: Messgerät
- 26: Optikmodul
- 27: Magazin
- 28: Andruckplatte
- 29: Gehäuse
- 30: Auswerfer
- 31: Auswurfkanal
- 32: Deckel
- 33: Stössel für analytisches Hilfsmittel
- 34: Mulde für Finger
- 35: Öffnung für Stössel 33
- 36: Öffnung für analytisches Hilfsmittel 1

In Figur 1 ist eine bevorzugte Ausführungsform des erfindungsgemäßen analytischen Hilfsmittels (1) dargestellt. Figur 1 a zeigt in einer perspektivischen Aufsicht das analytische Hilfsmittel (1), teilweise im Zusammenspiel mit Bestandteilen eines Messgeräts (Führungsplatte (4); Lanzettenstössel (5)). Zur Verdeutlichung der Struktur der Führungsplatte (4) ist diese in Figur 1 a (und in den nachfolgenden Figuren 1 b und 1 c) rechts seitlich aus ihrer eigentlichen Position zur Seite geklappt dargestellt. Die seitlich weggeklappte Führungsplatte (4) ist selbstverständlich in der realen Ausführungsform des analytischen Hilfsmittels (1) bzw. des dazugehörigen Messgeräts entsprechend der linken Führungsplatte (4) montiert.

Das analytische Hilfsmittel (1) besteht im wesentlichen aus einem Testelement (2) und einer Lanzette (3). Das Testelement (2) besteht aus einem Rahmenelement (7), welches in der bevorzugten Ausführungsform, die hier abgebildet ist, ein im wesentlichen rechteckiger flacher Spritzgussformkörper aus Kunststoff ist. Auf der Oberseite des Rahmenelements (7) liegt zentral ein rechteckiges Nachweiselement (6), das von einer Zone eines saugfähigen Materials zur Aufnahme überschüssiger Probenflüssigkeit (Waste-Zone (8)) umgeben ist. Durch das Rahmenelement (7) und das Nachweiselement (6) verläuft senkrecht zur Ebene des Nachweiselements (6) die Durchtrittsöffnung (9) für die Lanzettennadel (11) der Lanzette (3).

Das Rahmenelement (7) weist an zwei gegenüberliegenden parallelen Außenkanten ein Profil auf, welches dazu dient, das analytische Hilfsmittel (1) im Zusammenwirken mit den Führungsnuten (17) der Führungsplatte (4) während der Bewegung des analytischen Hilfsmittels (1) in unterschiedliche Positionen des Messgeräts zu führen.

Das Rahmenelement (7) des Testelements (2) ist über ein Scharnier mit der Lanzette (3) verbunden. Die Lanzette (3) besteht ― wie insbesondere aus Figur 1 b ersichtlich ist ― aus dem Lanzettenkörper (10), der ebenfalls wie das Rahmenelement (7) ein Kunststoffformkörper ist, der in Spritzgusstechnik geformt wird, und einer Lanzettennadel (11), die vorzugsweise aus Metall ist und eine angeschliffene Spitze aufweist. Der Lanzettenkörper (10) weist an seinen seitlichen Rändern ein paar Führungsnocken (12) auf, die in entsprechende Führungsnuten (16) der Führungsplatte (4) eingreifen. Das Zusammenspiel von Führungsnut (16) und Führungsnocken (12) während der Bewegung des analytischen Hilfsmittels (1) im Messgerät bewirkt ein Herunterklappen der Lanzette (3) aus der Aufbewahrungsposition in die Stechposition.

Der Lanzettenkörper (10) enthält weiterhin Rückholfedern (13), die dazu dienen, die Lanzettennadel (11) nach erfolgter Stechbewegung in die Ausgangslage zurückzuholen.

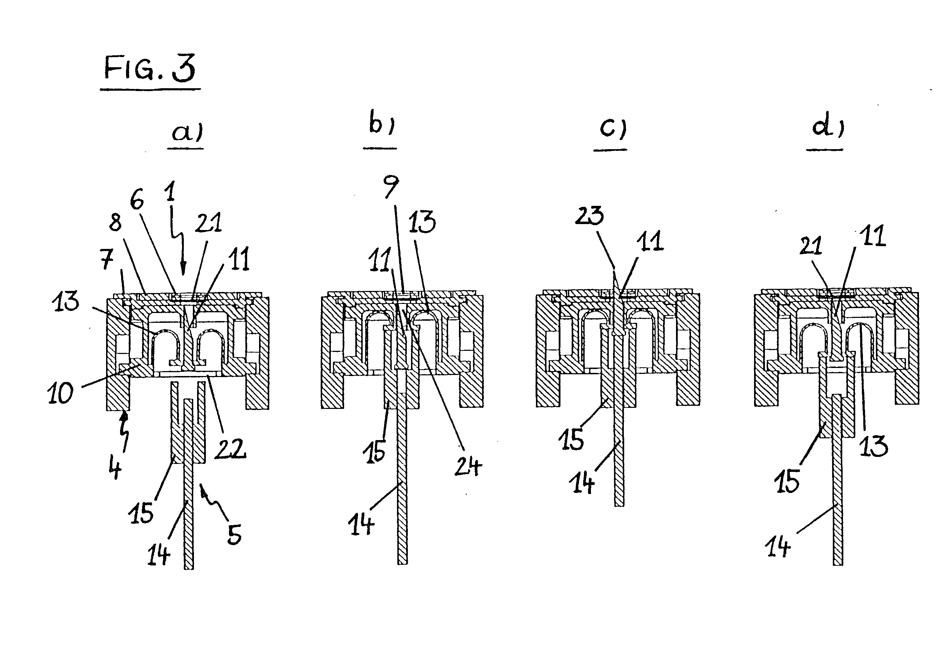

Aus Figur 1 b ist ebenfalls deutlich zu erkennen, wie der Lanzettenstössel (5) auf die Lanzette (3) einwirkt. Der Lanzettenstössel (5) enthält einen Stössel (14), der gezielt mit der Lanzettennadel (11) wechselwirkt und einem Stössel (15), der zum Vorspannen der Lanzettenrückholfeder (13) ausgestaltet ist. Das Zusammenwirken der einzelnen Stösselkomponenten mit der Lanzette (3) wird im Zusammenhang mit Figur 3 noch näher beschrieben werden.

In Figur 1 c ist eine Aufsicht auf das analytische Hilfsmittel (1) dargestellt. Insbesondere wird in dieser Ansicht nochmals deutlich, in welcher Reihenfolge die einzelnen Komponenten des Testelements (2) angeordnet sind: Eine zentrale Aussparung im Testelement (2) dient als Durchtrittsöffnung (9) für die Lanzettennadel. Die Durchtrittsöffnung (9) wird von einem im wesentlichen rechteckigen Nachweiselement (6) umgeben, welches wiederum unmittelbar an die es umgebende Waste-Zone (8), die beispielsweise aus einem Stück saugfähigen Papiers bestehen kann, umgeben ist. Nachweiselement (6) und Waste-Zone (8) sind auf einer Auflagefläche des Rahmenelements (7) beispielsweise durch Kleben befestigt.

Die in der Aufsicht von Figur 1 c abgebildete Oberfläche des analytischen Hilfsmittels (1) stellt die Fläche dar, die im Betrieb des analytischen Hilfsmittels (1) mit der Hautoberfläche des zu untersuchenden Individuums in Berührung kommt. Das zu untersuchende Individuum legt beispielsweise den Finger auf die obere Oberfläche des analytischen Hilfsmittels (1). Die Dimensionen des analytischen Hilfsmittels (1) sind so gewählt, dass vorzugsweise die Fläche des Nachweiselements (6) und der Waste-Zone (8) vom Finger des zu untersuchenden Individuums bedeckt werden können. Beim Stechvorgang durch die Lanzette (3), die vom Lanzettenstössel (5) angetrieben wird, durchdringt die Lanzettennadel (11) die Durchtrittsöffnung (9) und dringt in die Haut des zu untersuchenden Individuums ein. Nach dem Zurückziehen der Lanzettennadel (11) verbleibt die durchstochene Hautpartie des zu untersuchenden Individuums in unveränderter relativer Lage zum analytischen Hilfsmittel (1). Die durch die Lanzettennadel (11) erzeugte Wunde lässt einen Blutstropfen auf der Hautoberfläche entstehen, der vom Nachweiselement (6) aufgenommen wird. Eventuell überschüssiges Blut wird von der Waste-Zone (8) aufgenommen. Durch die geometrische Anordnung von Durchtrittsöffnung (9) und Nachweiselement (6) ist es möglich, dass die Blutprobe quasi im Moment des Entstehens auf das Nachweiselement (6) aufgebracht wird.

In Figur 2 ist anhand von 4 Teilfiguren in Seitenansicht dargestellt, wie das analytische Hilfsmittel (1) aus Figur 1 mit der Führungsplatte (4) beim Transport des analytischen Hilfsmittels (1) aus einer Aufbewahrungsposition in die Mess- bzw. Stechposition und anschließend aus dieser Mess- bzw. Stechposition wechselwirkt.

In Figur 2 a ist der Beginn des Transports des analytischen Hilfsmittels (1) aus der Aufbewahrungsposition in die Messposition abgebildet. Die Aufbewahrungsposition, in der die Lanzette (3) im Wesentlichen vollständig innerhalb der Begrenzungsflächen des Testelements (2) liegt, zeichnet sich dadurch aus, dass die Lanzettennadel (11) im Wesentlichen parallel zur Ebene des Testelements (2), insbesondere zur Ebene des Nachweiselements (6), liegt. Diese Position ist in Figur 2 a zu sehen. Die Messposition entspricht dem Zustand, der von dem analytischen Hilfsmittel (1) eingenommen wird, wenn die Führungsnocken (12) der Lanzette (3) im senkrechten Abschnitt der Führungsnut (16) angelangt sind. Die Lanzette (3) ist in diesem Zustand genau dem Lanzettenstössel (5) (bestehend aus Nadelstössel (14) und Federstössel (15)) gegenüberliegend angeordnet (vgl. auch Fig. 2 c). Die Lanzettennadel (11) ist in diesem Fall im Wesentlichen senkrecht zur Ebene des Testelements (2), insbesondere zur Ebene des Nachweiselements (6), orientiert, wobei die Nadelspitze in Richtung des Testelements (2) weist.

Wie im einzelnen aus den Figuren 2 a bis 2 d ersichtlich ist, wird beim Transport des analytischen Hilfsmittels (1) in Richtung (19) das Rahmenelement (7) des Testelements (2) durch die Führungsnut (17) geführt. Die Transportrichtung (19) verläuft durch die Führung geradlinig waagrecht. Die gekrümmte Führungsnut (16) sorgt im Wechselspiel mit den Nocken (12) dafür, dass im Verlauf der linearen Bewegung des analytischen Hilfsmittels (1) der Lanzettenkörper (10) um das Scharnier (18) in Richtung (20), d. h. im wesentlichen nach unten geklappt wird, bis die Mess- bzw. Stechposition, die in Figur 2 c dargestellt ist, erreicht ist. In diesem Zustand verbleibt das analytische Hilfsmittel (1) so lange, bis der Stech- bzw. Messvorgang abgeschlossen ist. Die Lanzette (3) befindet sich hierbei in der Stechposition, die sich von der in Figur 2 a dargestellten Aufbewahrungsposition der Lanzette (3) dadurch unterscheidet, dass die Lanzettennadel (11) im wesentlichen orthogonal zur Ebene des Testelements (2) orientiert ist.

Nach der Durchführung der Messung wird ― wie in Figur 2 d dargestellt ist ― das erfindungsgemäße analytische Hilfsmittel (1) weiter in Richtung (19) transportiert. Die Führungsnut (16) sorgt durch ihren gekrümmten Charakter dafür, dass die Lanzette (3) in Richtung (20) zurück in die Aufbewahrungsposition klappt. Die Lanzettennadel (11) ist in dieser Aufbewahrungsposition im endgültigen Zustand wieder parallel zur Ebene des Testelements (2) orientiert.

In Figur 3 ist in vier Teilfiguren (Figur 3 a ― 3 d) schematisch das Zusammenwirken des Lanzettenstössels (5) mit der Lanzette (3) des analytischen Hilfsmittels (1) aus Figur 1 und 2 abgebildet. Figur 3 a zeigt das analytische Hilfsmittel (1) in der Mess- und Stechposition, die im wesentlichen Figur 2 c entspricht. Nadelstössel (14) und Federstössel (15) des Lanzettenstössels (5) sind in der in Figur 3 a abgebildeten Ausgangsposition unterhalb der Lanzette (3) angeordnet. Die Lanzette (3) ist noch in der Ruhelage der Stechposition. Die Lanzettennadel (11) wird von der Rückholfeder (13) in der Ruhelage gehalten. Der Lanzettenkörper (10) fixiert im Zusammenspiel mit der Führungsnut (16) die Position der Lanzette (3) und sorgt somit für eine eindeutige Ausrichtung der Lanzettennadel (11) relativ zum Lanzettenstössel (5) einerseits und zum Testelement (2), insbesondere zum Nachweiselement (6), andererseits. In Figur 3 a ist deutlich zu erkennen, dass das Testelement (2), welches aus dem Rahmenelement (7) und den schichtenförmigen Bestandteilen Nachweiselement (6) und Waste-Zone (8), die auf der Auflagefläche des Rahmenelements befestigt sind, im Bereich der Durchtrittsöffnung (9) eine Gummimembran als Septum (21) aufweist. Dieses kann beim Stechvorgang von der Lanzettennadel (11) durchdrungen werden und sich nach dem Zurückziehen der Lanzettennadel (11) in die Ausgangsposition wieder dicht verschließen.

Wie ebenfalls aus den Figuren 3 a bis 3 d ersichtlich ist, weist der Lanzettenkörper (10) eine Durchtrittsöffnung (22) für den Lanzettenstössel (5) auf. Diese liegt auf der dem stumpfen Ende der Lanzettennadel (11) zugewandten Seite. Die Spitze (23) der Lanzettennadel (11) wird im Ruhezustand von einer Führungshülse (24) umgeben, die ebenfalls Teil des Lanzettenkörpers (10) ist. Zusätzlich dient die Führungshülse (24) dazu, die Spitze (23) zu umschließen, um ein unbeabsichtigtes Verletzen an der Lanzettennadel (11) zu vermeiden.

In Figur 3 b ist dargestellt, wie zu Beginn des Stechvorgangs zunächst der Federstössel (15) auf den die Lanzettennadel (11) haltenden Teil des Lanzettenkörpers (10) einwirkt und so die Rückholfeder (13) in den gespannten Zustand bewegt. In Figur 3 c ist dargestellt, wie im Anschluss an die Vorspannung der Rückholfeder (13) durch den Federstössel (15) der Nadelstössel (14) auf die Lanzettennadel (11) einwirkt und diese mit der Spitze (23) durch das Septum (21) hindurchbewegt, so dass die Spitze (23) der Lanzettennadel (11) aus der Oberfläche des analytischen Hilfsmittels (1) herausragt und durch die Haut eines zu untersuchenden Individuums eindringen kann. Die Stechbewegung der Lanzettennadel (11) wird dabei durch die Führungshülse (24) des Lanzettenkörpers (10) und die Durchtrittsöffnung (9) des Testelements (2) geführt.

In Figur 3 d ist schematisch dargestellt, wie sich nach dem Zurückziehen des Lanzettenstössels (5), das heisst des Nadelstössels (14) und des Federstössels (15), die Rückholfeder (13) die Lanzettennadel (11) in die Ausgangsposition zurückbewegt. Dabei schließt sich das Septum (21). Die Spitze (23) der Lanzettennadel (11) liegt nun wieder vollständig innerhalb des Lanzettenkörpers (10). Das wiederverschlossene Septum (21) sorgt dafür, dass Blut, das sich nach dem Stechvorgang auf der oberen Oberfläche des Testelements (2) sammelt, nicht auf die Unterseite des Testelements (2) durchdringen und somit potentiell Teile des Messgeräts verschmutzen kann.

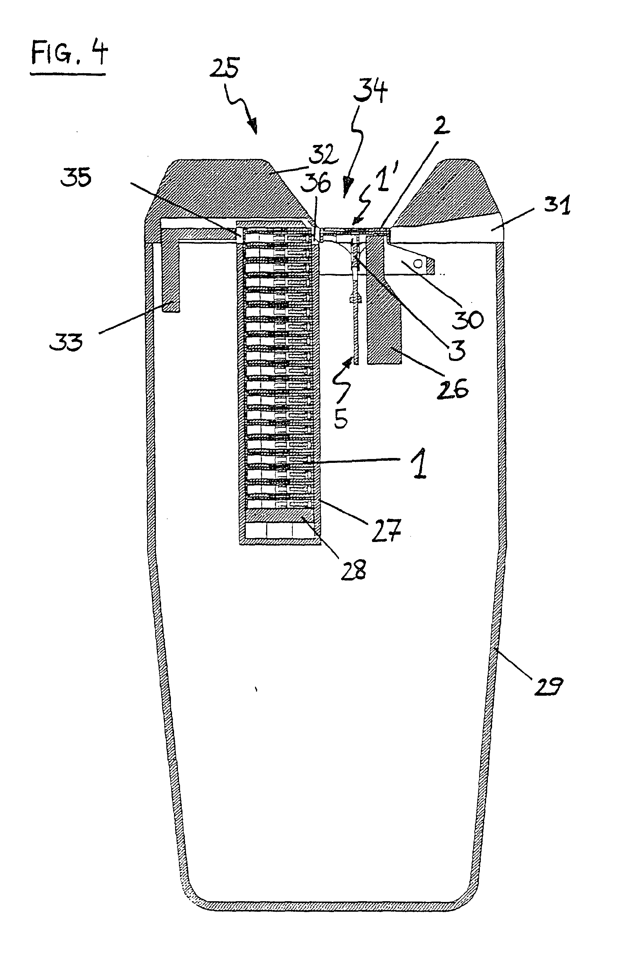

In Figur 4 ist eine bevorzugte Ausführungsform eines Messgeräts (25) in einer Längsschnittansicht abgebildet. Das Messgerät (25) enthält ein Magazin (27) zur Aufbewahrung von analytischen Hilfsmitteln (1), wie sie in besonders bevorzugten Ausführungsformen im Zusammenhang mit Figur 1 bis 3 beschrieben wurden. Das Magazin (27) kann im Bedarfsfall durch Abnehmen des Deckels (32) vom Messgerät (25) entnommen und durch ein neues Magazin ersetzt werden. Deckel (32) ist zu diesem Zweck vorzugsweise mit Hilfe eines Scharniers am Messgerätegehäuse (29) befestigt.

Das Messgerät (25) enthält weiterhin einen Stössel (33), mit dessen Hilfe analytische Hilfsmittel (1) aus dem Magazin in die Mess- bzw. Stechposition befördert werden können. Das Magazin (27) weist zu diesem Zweck eine Öffnung (35) auf, durch die der Stössel (33) eindringen kann, und besitzt eine der Öffnung (35) gegenüberliegende Öffnung (36), durch die das analytische Hilfsmittel (1) aus dem Magazin (27) in die Messposition gelangen kann. Analytische Hilfsmittel (1), aus dem im Magazin befindlichen Vorrat, können durch eine Andruckplatte (28), die entweder Teil des Magazins (27) oder integrierter Teil des Messgeräts (25) sind, an die Position des entnommenen analytischen Hilfsmittels (1) im Magazin (27) nachgerückt werden. Die Andruckplatte (28) kann von einem manuell zu bedienenden Schieber, einem Motor oder einer Feder angetrieben werden.

In Figur 4 ist ein analytisches Hilfsmittel (1') in der Mess- bzw. Stechposition gezeigt. Die Lanzette (3) ist dabei ausgeklappt, so dass sie im wesentlichen senkrecht zur Ebene des Testelements (2) liegt. Der Lanzette (3) gegenüberliegend befindet sich in der Mess- bzw. Stechposition der Lanzettenstössel (5). Zur Auswertung des Nachweiselements besitzt das Messgerät (25) in der bevorzugten Ausführungsform ein bewegliches Optikmodul (26), welches für den Transport des analytischen Hilfsmittels (1) abgesenkt werden kann. Das Vermessen des Testelements mit dem Optikmodul (26) erfolgt nach an sich bekannten Verfahren, beispielsweise reflexionsfotometrisch.

Das Messgerät (25) enthält weiterhin einen Auswerfer (30), der im Zusammenspiel mit dem Rahmenelement (7) des analytischen Hilfsmittels (1) dieses nach erfolgter Messung über den Auswurfkanal (31) aus dem Messgerät (25) entfernt. Alternativ kann das benutzte analytische Hilfsmittel (1) in ein weiteres Magazin (nicht gezeigt) zur Aufbewahrung und Entsorgung überführt werden.

Das Messgerät (25) weist eine Mulde oder Rinne (34) auf, in die beispielsweise ein Finger eines zu untersuchenden Individuums gelegt werden kann. Der Finger kann in dieser Mulde (34) mit dem analytischen Hilfsmittel (1') in Kontakt gebracht werden. Er kann während des gesamten Stech- und Messvorgangs in unveränderter Position behalten werden.

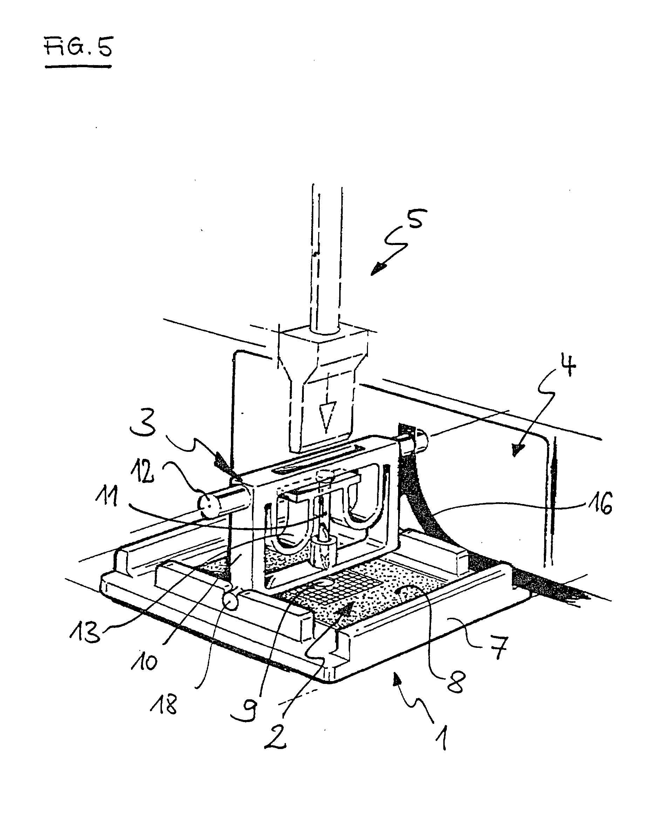

In Figur 5 ist eine weitere bevorzugte Ausführungsform des erfindungsgemäßen analytischen Hilfsmittels (1) abgebildet. Das analytische Hilfsmittel (1) aus Figur 5 entspricht dabei im wesentlichen dem analytischen Hilfsmittel (1) wie es in Figur 1 dargestellt ist. Es enthält eine Lanzette (3), die über ein Scharnier (18) mit dem Rahmen (7) eines Testelements (2) beweglich verbunden ist. Schematisch ist in Figur 5 auch der Lanzettenstössel (5) dargestellt, der während einer Stechbewegung mit der Lanzette (3) des analytischen Hilfsmittels (1) zusammenwirkt. Funktion und Bezeichnung der übrigen Elemente des analytischen Hilfsmittels (1) aus Figur 5 sind im wesentlichen identisch mit denen des analytischen Hilfsmittels (1) aus Figur 1. Auf diese Figur wird hier ausdrücklich Bezug genommen.

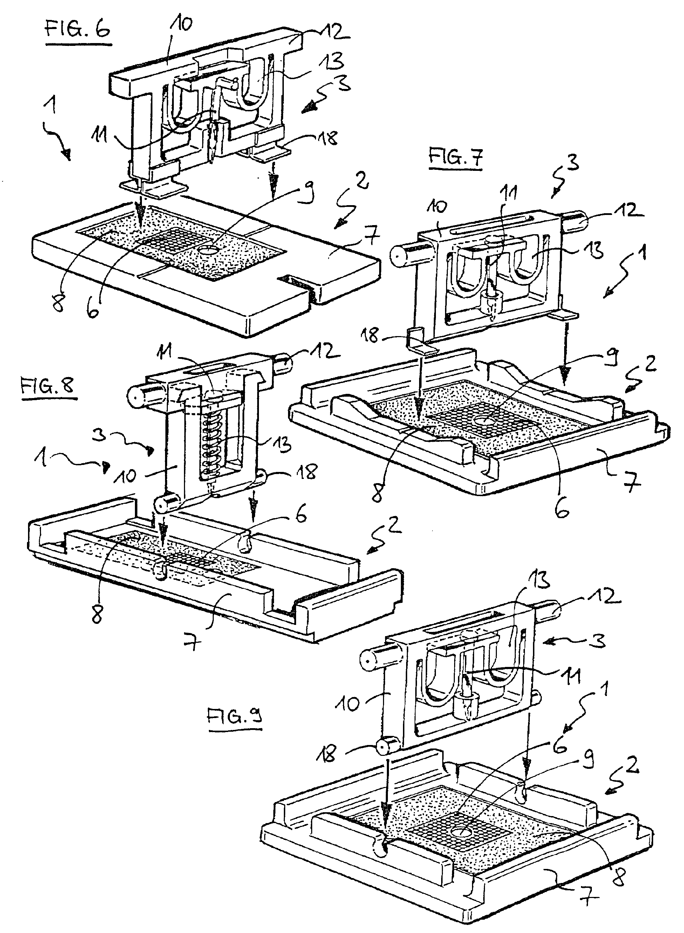

In den Figuren 6 bis 9 sind alternative, ebenfalls bevorzugte Ausführungsformen des erfindungsgemäßen analytischen Hilfsmittels (1) in teilweiser Explosionsansicht dargestellt. Die analytischen Hilfsmittel (1) der Figuren 6 bis 9 enthalten jeweils eine Lanzette (3), die über ein Scharnier oder Gelenk (18) mit einem Testelement (2) verbunden ist. In den Figuren 6 bis 9 sind jeweils die Lanzetten (3) und die Testelemente (2) getrennt voneinander dargestellt. Die Pfeile in diesen Figuren deuten daraufhin, wie die beiden Elemente (Lanzette (3) und Testelement (2)) beispielsweise während der Fertigung zusammengebracht werden können.

Die analytischen Hilfsmittel (1) aus Figur 6 bis 9 entsprechen im wesentlichen den analytischen Hilfsmitteln (1), wie sie in den Figuren 1 und 5 bereits beschrieben sind. Auf die Beschreibung dieser Figuren wird hier ausdrücklich Bezug genommen.

Das analytische Hilfsmittel (1) aus Figur 6 ist im wesentlichen aus folien- bzw. bandförmigen Materialien aufgebaut. Insbesondere der Rahmen (7) des Testelements (2) und der Lanzettenkörper (10) der Lanzette (3) sind aus Folienmaterial hergestellt. Auch das Scharnier (18) ist aus Folienmaterial gefertigt. Lanzette (3) und Testelement (2) werden über das Foliengelenk (18) miteinander verbunden. Die Verbindung kann beispielsweise durch Verkleben oder Verschweißen erfolgen.

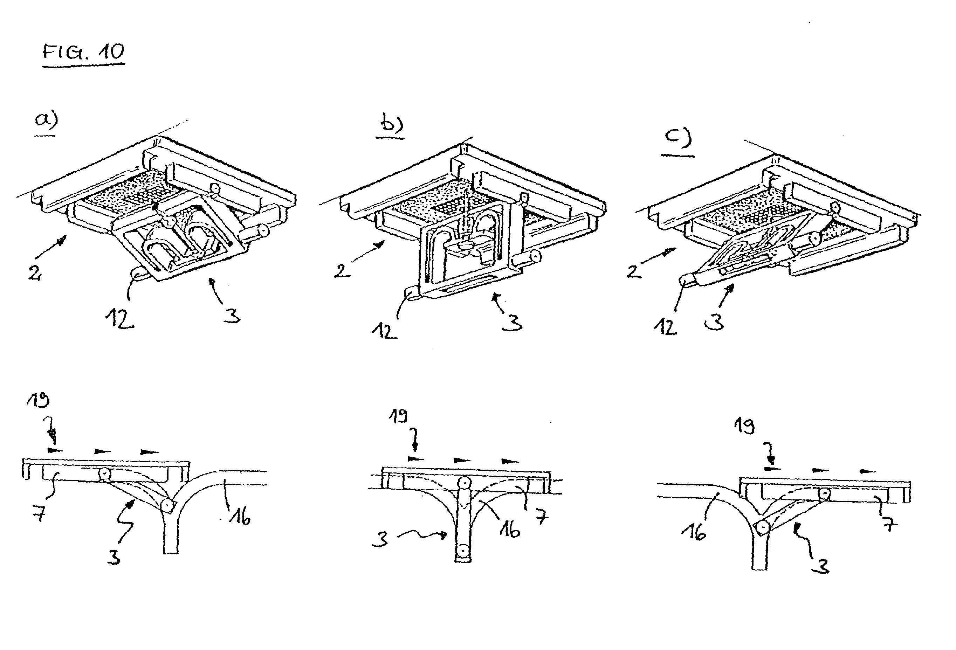

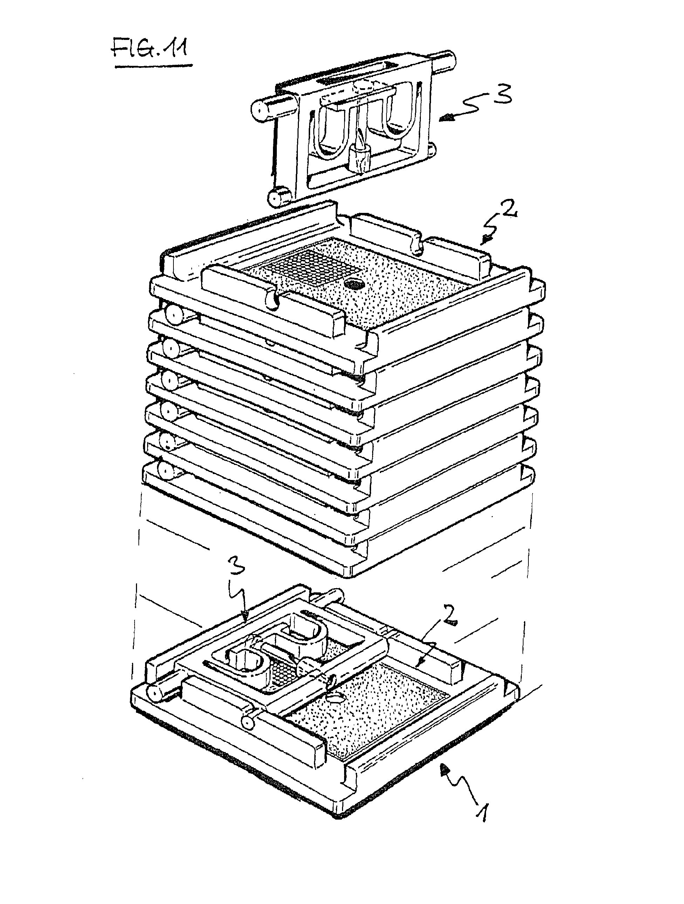

In den Figuren 7 bis 9 sind analytische Hilfsmittel (1) abgebildet, bei denen der Rahmen (7) des Testelements (2) und der Lanzettenkörper (10) der Lanzette (3) aus Spritzgussteilen besteht. Bei diesen analytischen Hilfsmitteln (1) können die Lanzette (3) und das Testelement (2) über ein Folienscharnier (18), wie in Figur 7 gezeigt, oder aber - wie in Figur 8 und 9 gezeigt - über ein Gelenk (18), das im wesentlichen aus einem Paar zylindrischer Zapfen, die in entsprechende Ausnehmungen des Rahmens (7) des Testelements (2) eingreifen, miteinander verbunden sein.

Wie im Vergleich der Figuren 6, 7 und 9 mit Figur 8 ersichtlich ist, kann die Rückholfeder (13) der Lanzette (3) unterschiedlich ausgestaltet sein. Während die Rückholfeder (13) in Figuren 6, 7 und 9 einstückig mit dem Lanzettenkörper 10 ausgebildet ist, wird in Figur 8 eine Schraubenfeder (13), die vorzugsweise aus Metall gefertigt ist, dargestellt.

In Figur 10 werden in perspektivischer Darstellung drei Momentaufnahmen (Figur 10a, b und c) der Bewegung des analytischen Hilfsmittels (1) beim Transport von der Aufbewahrungs- in bzw. aus die Messposition gezeigt. Die obere Teilfigur ist dabei jeweils eine perspektivische Aufsicht auf die Unterseite des analytischen Hilfsmittels, während die untere Teilfigur eine vereinfachte Seitenansicht darstellt. Die in den Figuren 10a bis c dargestellten Stadien entsprechen denjenigen, die in den Figuren 2b bis d abgebildet sind. Für Details wird auf die Beschreibung von Figur 2 weiter oben verwiesen.

In Figur 11 ist schematisch in einer perspektivischen Aufsicht auf die Unterseite ein Stapel von analytischen Hilfsmitteln (1), wie sie näher in Figur 5 beschrieben sind, abgebildet. Ein solcher Stapel von analytischen Hilfsmitteln (1) befindet sich beispielsweise im Magazin (27) des Messgeräts (25) aus Figur 4.

Das unterste analytische Hilfsmittel (1) in Figur 11 befindet sich in der Aufbewahrungsstellung. Dabei ist die Lanzette (3) in den Rahmen des Testelements (2) eingeklappt. Die Lanzettennadel liegt dabei im wesentlichen parallel zur Ebene des Testelements.

Im obersten analytischen Hilfsmittel (1) in Figur 11 sind die Lanzette (3) und das Testelement (2) nochmals separat gezeigt. Selbstverständlich sind in Wirklichkeit die Lanzette (3) und das Testelement (2) über das Scharnier miteinander verbunden. Im Gegensatz zum untersten analytischen Hilfsmittel (1) aus Figur 11 ist im obersten analytischen Hilfsmittel (1) in Figur 11 die Lanzette (3) im wesentlichen senkrecht zur Ebene des Testelements (2) orientiert. Dies entspräche der Orientierung dieser beiden Bestandteile des analytischen Hilfsmittels (1) in Stechposition.

## Patentansprüche

1. Analytisches Hilfsmittel (1) für die Gewinnung und Untersuchung einer Körperflüssigkeit, enthaltend ein Testelement (2) und eine Lanzette (3), wobei
(i) das Testelement (2) folgende Komponenten enthält:
- ein Rahmenelement (7);
- zumindest ein Nachweiselement (6), das direkt oder indirekt mit dem Rahmenelement (7)verbunden ist; und
(ii) die Lanzette (3) folgende Komponenten enthält:
- eine Nadel (11) mit Spitze (23);
- einen Lanzettenkörper (10), der die Nadel (11) zumindest teilweise umgibt; **dadurch gekennzeichnet, dass** der Lanzettenkörper (10) beweglich mit dem Rahmenelement (7) des Testelements (2) verbunden ist, so dass die Lanzette (3) eine Aufbewahrungsposition und eine von der Aufbewahrungsposition verschiedene Stechposition einnehmen kann, wobei die Nadel (11) in der Aufbewahrungsposition im wesentlichen parallel zur Ebene des Testelements (6) und in der Stechposition im wesentlichen orthogonal zur Ebene des Testelements (6) orientiert ist.

2. Analytisches Hilfsmittel (1) gemäß Anspruch 1, **dadurch gekennzeichnet, dass** das Testelement (2) in unmittelbarer Nachbarschaft zum Nachweiselement (6) eine separate Zone (8) aus saugfähigem Material enthält, die zur Aufnahme überschüssiger Probenflüssigkeit geeignet ist.

3. Analytisches Hilfsmittel (1) gemäß einem der vorhergehenden Ansprüche, **dadurch gekennzeichnet, dass** das Nachweiselement (6) des Testelements (2) und gegebenenfalls die Zone (8) aus saugfähigem Material auf einer gemeinsamen Trägerschicht befestigt sind.

4. Analytisches Hilfsmittel (1) gemäß einem der vorhergehenden Ansprüche, **dadurch gekennzeichnet, dass** der Lanzettenkörper (10) über zumindest ein Scharnier (18) oder Gelenk mit dem Rahmenelement (7) des Testelements (2) beweglich verbunden ist.

5. Analytisches Hilfsmittel (1) gemäß einem der vorhergehenden Ansprüche, **dadurch gekennzeichnet, dass** das Rahmenelement (7) des Testelements (2) und der Lanzettenkörper (10) Kunststoffspritzgussteile sind.

6. Analytisches Hilfsmittel (1) gemäß einem der vorhergehenden Ansprüche, **dadurch gekennzeichnet, dass** das Testelement (2) eine Durchtrittsöffnung (9) für die Lanzettennadel (11) aufweist.

7. Analytisches Hilfsmittel (1) gemäß Anspruch 6, **dadurch gekennzeichnet, dass** die Durchtrittsöffnung (9) im Bereich des Nachweiselements (6) liegt.

8. Analytisches Hilfsmittel (1) gemäß einem der vorhergehenden Ansprüche, **dadurch gekennzeichnet, dass** der Lanzettenkörper (10) eine Rückholfeder (13) für die Lanzettennadel (11) enthält.

9. Analytisches Hilfsmittel (1) gemäß einem der vorhergehenden Ansprüche, **dadurch gekennzeichnet, dass** der Lanzettenkörper (10) und/oder das Rahmenelement (7) eine Führungshülse (24) für die Lanzettennadel (11) aufweisen.

10. Analytisches Hilfsmittel (1) gemäß einem der vorhergehenden Ansprüche, **dadurch gekennzeichnet, dass** das Rahmenelement (7) ein Führungsprofil an zwei gegenüberliegenden parallelen Seiten aufweist.

11. Analytisches Hilfsmittel (1) gemäß einem der vorhergehenden Ansprüche, **dadurch gekennzeichnet, dass** der Lanzettenkörper (10) ein Paar außenliegender Nocken (12) aufweist.

12. System zur Bevorratung von analytischen Hilfsmitteln (1) umfassend ein Magazin (27) und einen Vorrat an analytischen Hilfsmitteln (1) gemäß einem der vorhergehenden Ansprüche.

13. System gemäß Anspruch 12, **dadurch gekennzeichnet, dass** das Magazin (27) komplementäre Führungsprofile für das Rahmenelement (7) des analytischen Hilfsmittels (1) gemäß Anspruch 10 enthält.

14. System gemäß Anspruch 12 oder 13, **dadurch gekennzeichnet, dass** das Magazin (27) Führungsnute für die Nocken (12) des Lanzettenkörpers (10) des analytischen Hilfsmittels (1) gemäß Anspruch 11 enthält.

15. System zur Bestimmung der Anwesenheit oder des Gehalts eines Analyten in Blut, umfassend ein Messgerät (25) zum Messen und Anzeigen der Änderung einer mit dem Analyten korrelierten, charakteristischen Eigenschaft eines Testelements (2) und ein Magazin (27) geeignet zur Aufnahme von analytischen Hilfsmitteln (1) gemäß einem der Ansprüche 1 bis 11 ist.

16. System gemäß Anspruch 15, **dadurch gekennzeichnet, dass** das Messgerät (25) komplementäre Führungsprofile (17) für das Rahmenelement (7) des analytischen Hilfsmittels (1) gemäß Anspruch 10 enthält.

17. System gemäß Anspruch 15 oder 16, **dadurch gekennzeichnet, dass** das Messgerät (25) Führungsnute (16) für die Nocken (12) des Lanzettenkörpers (10) des analytischen Hilfsmittels (1) gemäß Anspruch 11 enthält.

18. System gemäß Anspruch 15, **dadurch gekennzeichnet, dass** das Messgerät (25) eine Vorrichtung (5) zum Antrieb der Lanzette (3) des analytischen Hilfsmittels (1) gemäß Anspruch 1 umfasst.

19. Klappbare Lanzette umfassend ein Rahmenelement (7) und eine Lanzette (3), wobei die Lanzette (3) eine Nadel (11) mit Spitze (23) und einen die Lanzettennadel (11) teilweise umgebenden Lanzettenkörper (10) enthält, **dadurch gekennzeichnet, dass** der Lanzettenkörper (10) beweglich mit dem Rahmenelement (7) verbunden ist, so dass die Lanzette (3) eine Aufbewahrungsposition und eine von der Aufbewahrungsposition verschiedene Stechposition einnehmen kann, wobei die Nadel (11) in der Aufbewahrungsposition im wesentlichen parallel zur Ebene des Rahmenelements (7) und der Stechposition im wesentlichen orthogonal zur Ebene des Rahmenelements (7) orientiert ist.
